# EUROPEAN PATENT APPLICATION

(11) **EP 1 836 946 A1**
(43) Date of publication of application: **26.09.2007**
(21) Application number: 07005794.8
(22) Date of filing: 21.03.2007
(51) Int. Cl.: A61B 1/00, A61B 1/04, A61B 1/05

(54) **Endoscopic apparatus**

(30) Priority: 22.03.2006 JP 2006078046; 20.03.2007 JP 2007073046
(71) Applicant: Fujinon Corporation, Saitama-shi, Saitama (JP)
(72) Inventor: Purwanto, Eko, Saitama-shi Saitama (JP)
(74) Representative: Klunker . Schmitt-Nilson . Hirsch

(57) **Abstract**

An endoscopic apparatus comprises an endoscope including an objective optical system comprising a power-changing movable lens and a focusing movable lens so as to form a subject image and an imager that photoelectrically converts the subject image, to have an optical variable-power function that optically changes a magnification of the subject image through the power-changing movable lens and an electronic variable-power function that electronically enlarges the subject image taken by the imager. The focusing movable lens that makes a focal adjustment of the subject image at between: a first focusing point and a second focusing point on a nearest point side. The endoscopic apparatus further comprises an auto-focus circuit that performs, during operation with the electronic variable-power function, an auto-focusing on the subject image through use of the focusing movable lens by taking, as the first focusing point, a position of the power-changing movable lens upon shifting from optical power change to electronic power change.

## Description

### Background of the Invention

### 1. Field of the Invention

The present invention relates to endoscopic apparatuses, and more particularly to the auto-focus function to obtain an enlarged video image of a subject by optical and electronic power-change functions and perform a focal adjustment of an enlarged subject image, and to a control content thereof.

### 2. Description of the Related Art

The electronic endoscopic apparatus is made up with an electronic endoscope (scope), a processor unit, a light source, a monitor and so on. The electronic endoscope has an insert-part tip arranging therein an objective optical system and a CCD (charge coupled device) serving as a solid-state imager, and so on. Based on the illumination of from the light source, a subject is imaged by the objective optical system and the CCD. The image signal is subjected to a video processing in the processor unit, thereby displaying a video image of the subject on the monitor.

In the endoscopic apparatus of this kind, the subject image can be optically changed in magnification by incorporating an optical power-change mechanism (zoom mechanism) having a movable lens in the objective optical system, as disclosed in JP-A-2002-277756. Meanwhile, image-enlargement process is performed on the video-signal data obtained in the CCD etc., to obtain an electronically-enlarged video image of the subject. According to such optical variable-power mechanism (function) and electronic variable-power function, an enlarged image can be viewed with quality by further electronically enlarging the image once taken optically at a great magnification.

Meanwhile, there is a proposed endoscopic apparatus incorporating therein an auto-focus mechanism to adjust the focal point of the optical lens system while varying the focal length in accordance with the distance to a subject, as disclosed in JP-A-2002-263058.

In the meanwhile, the endoscopic apparatus principally is to take an image of a fine tissue, e.g. a diseased point, as a subject to observe. This requires the auto-focus function to operate well where the subject being observed is taken at a short distance or such a subject is to be enlarged. In addition, when to enlarge the subject image, particularly when further electronically enlarging the video image once optically taken at a great magnification, focal adjustment must be done with precision in order to avoid conspicuous blur in the image.

### Summary of the Invention

The present invention has been made in view of the foregoing problem, and it is an object thereof to provide an endoscopic apparatus that precise focal adjustment is possible to perform even where further electronically enlarging the video image optically taken at a great magnification thereby enabling to observe, in well state, an electronically enlarged video image of a subject under observation.

In order to achieve the foregoing object, a first aspect of the invention is an endoscopic apparatus comprising an endoscope, the endoscope comprising: an objective optical system, provided in a distal end of the endoscope, comprising a power-changing movable lens and a focusing movable lens so as to form a subject image; and an imager that photoelectrically converts the subject image, so that the endoscopic apparatus has an optical variable-power function that optically changes a magnification of the subject image through the power-changing movable lens and an electronic variable-power function that electronically enlarges the subject image taken by the imager, wherein the focusing movable lens makes a focal adjustment of the subject image at between: a first focusing point on a near point side from a distant point, the first focusing point being established on the power-changing movable lens having the optical variable-power function; and a second focusing point on a nearest point side, and wherein the endoscopic apparatus further comprises an auto-focus circuit that performs, during operation with the electronic variable-power function, an auto-focusing on the subject image through use of the focusing movable lens by taking, as the first focusing point, a position of the power-changing movable lens upon shifting from optical power change to electronic power change.

According to a second aspect of the invention, the endoscope further comprises an endoscopic operation part including a power-change instructing section (power-change switch) that instructs a power change to continuously perform a power change while switching over between the optical variable-power function and the electronic variable-power function, so as to perform an auto-focusing during operation with the electronic variable-power function.

According to a third aspect of the invention, the auto-focus circuit establishes focus-detection areas (in an image area established by the optical variable-power function in a shift to electronic power change) smaller in area correspondingly to an increasing magnification of electronic power change.

According to the above structure, the power-change instructing section, optical and electronic power changes and shift between the both power changes are operated, say, by the power-changing instructing section. Due to this, a subject image can be obtained by further electronically enlarging the image taken optically at a great magnification. During the optical power change, optical power change is made by moving the power-changing movable lens. Simultaneously, focal point moves from a distant position to an intermediate focusing position (position where shifted to electronic power change). During electronic power change, auto-focusing is made in the range of from the intermediate focusing position up to the nearest point. Accordingly, in an enlarged video image based on electronic power change, well image quality is obtained owing to precise auto-focusing.

Meanwhile, in the third aspect of the invention, the focus-detecting areas are established in smaller areas (focus-detecting areas equal in size or further smaller than those of enlarged-video-image display areas) correspondingly to the higher magnification of electronic power change. Accordingly, a in-focus state is detected in an area adapted for an enlarged video image, thus favorably effecting auto-focusing.

### Brief Description of the Drawings

Fig. 1 is a diagram showing the main arrangement of an electronic endoscopic apparatus according to an embodiment of the present invention;
Fig. 2 is a sectional view showing an interior structure of an endoscope tip in the embodiment;
Fig. 3 is a view showing an exterior arrangement of the entire of the electronic endoscopic apparatus;
Fig. 4 is a figure showing the operation of first to third movable lenses of the embodiment, in terms of a relationship between a distance to a subject and a lens movement amount;
Fig. 5 is a figure showing focus-detecting areas (within image area) established under auto-focus control in the embodiment;
Figs. 6A to 6C are figures showing a focus-detecting area (within display area) established under auto-focus control in the embodiment and a best in-focus position; and
Fig. 7 is a flowchart showing an operation of the embodiment.

### Detailed Description of the Invention

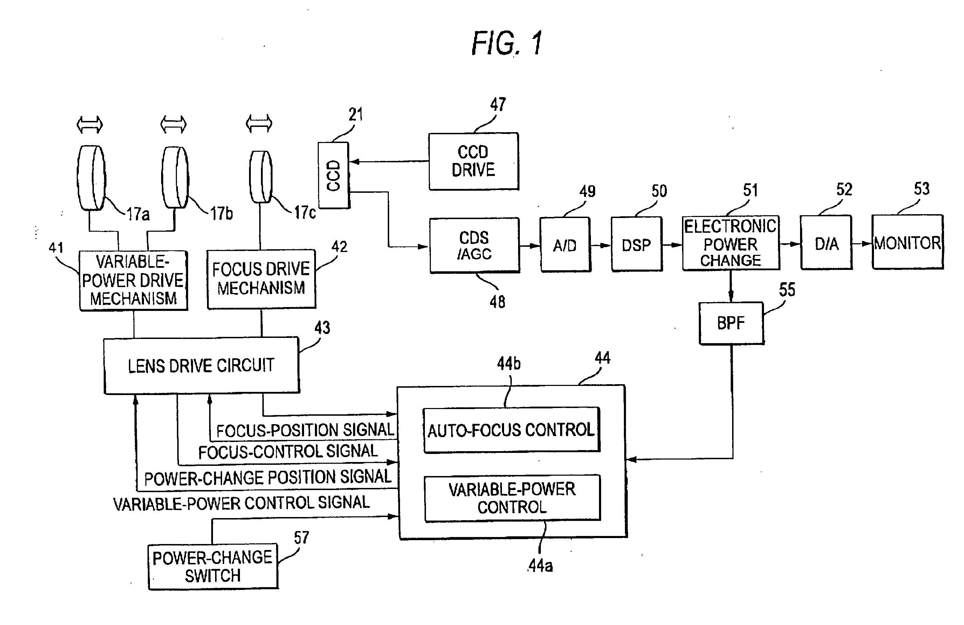

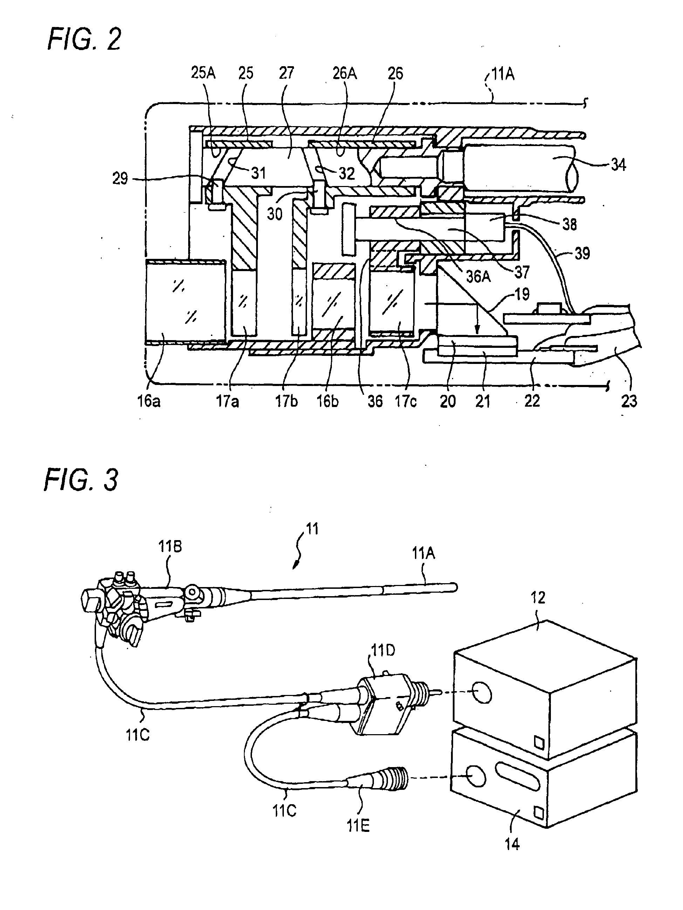

Fig. 1 shows a main arrangement of an electronic endoscopic apparatus according to an embodiment, Fig. 2 shows an example of an interior structure of the endoscopic tip thereof, and Fig. 3 shows an exterior structure of the electronic endoscopic apparatus. In Fig. 3, the electronic endoscopic apparatus is made up with an electronic endoscope (scope) 11 formed by an insertion part 11A to be inserted in a subject body and having an objective optical system and a solid-state imager such as a CCD at the tip thereof, an operation part 11B having an angle-operation knob and various operation switches etc., and a cable part 11C having a light-source connector 11D and an electric connector 11E, a light source 12 to which the electronic endoscope 11 is connected through the light-source connector 11D, a processor unit 14 to which the electronic endoscope 11 is connected through the electric connector 11E, and so on.

In Fig. 2, the insertion part 11A has a tip incorporating therein an objective optical system, i.e. a fixed lens 16a including a viewing window, power-changing first and second movable lenses 17a, 17b structured as a variable-focal lens capable of desirably changing the focal length, a fixed lens 16b and a third movable lens 17c for focal adjustment. In rear of the third movable lens 17c, a CCD 21 forming a solid-state imager is arranged though a prism 19 and a cover glass 20. The signal, taken an image of at the CCD 21, is delivered to the processor unit 14 through a circuit board 22 and a signal line 23.

The first movable lens 17a is held by a support (holder frame) 25 having an engagement hole 25A while the second movable lens 17b is by a support 26 having an engagement hole 26A. In the state the engagement holes 25A, 26A fit over an outer periphery of a cylindrical camshaft 27, the lenses 17a, 17b are arranged on the camshaft 27. A cam pin 29 is arranged projecting for the engagement hole 25A while a cam pin 30 is for the engagement hole 26A. In one end region of the camshaft 27, cam grooves 31, 32 are formed different in inclination direction relative to the axis thereof so that the cam pin 29 engages in the cam groove 31 while the cam pin 30 in the cam groove 32.

The camshaft 27 is coupled with a linear-transmission member 34 formed by a multi-coiled spring or the like. The linear-transmission member 34 has the other end connected to a motor provided in the operation part 11B. Accordingly, by rotating the camshaft 27 due to motor driving through the linear-transmission member 34, the first and second movable lenses 17a, 17b are moved back and forth in the optical-axis direction through the engagement of the cam grooves 31, 32 and the cam pins 29, 30. This causes a change of optical power (zooming). Namely, the first and second movable lenses 17a, 17b constitute a variable-focal optical system, to cause a change of optical power (making variable the observation distance, the observation depth, the focal length, etc.) while being moved back and forth.

Meanwhile, a focus drive mechanism (actuator) using a piezoelectric element is provided in order to drive the focusing, or third, movable lens 17c. The focus drive mechanism is arranged with a support (moving body) 36 serving as a holder frame sustaining the third movable lens 17c and formed with (cylindrical member having) an engagement hole 36A in the upper portion thereof, a cylindrical drive shaft (body) 37 frictionally engaged in the engagement hole 36A of the support 36, and a piezoelectric element 38 coupled (fixed) to the drive shaft 37. The piezoelectric element 38 is supplied with a drive pulse through the drive line 39. With the piezoelectric-element drive mechanism, by longitudinally moving the drive shaft 37 due to expansion/contraction driving of the piezoelectric element 38, the third movable lens 17c can be moved forward or backward. Incidentally, the power-changing first and second movable lenses 17a, 17b may be arranged to be driven by means of piezoelectric elements or the like.

In Fig. 1, the first and second movable lenses 17a, 17b are to be driven by means of a variable-power drive mechanism formed by the elements of from the Fig. 2 support 25 to the linear-transmission member 34 while the third movable lens 17c is to be driven by the focus drive mechanism 42 using the piezoelectric element 38. For the drive mechanisms 41, 42, a lens drive circuit 43 is provided to supply a drive signal for driving the lenses 17a - 17c. Furthermore, arranged is a control circuit (microcomputer, etc.) 44 having a variable-power control section 44a and an auto-focus control section 44. Meanwhile, for the CCD 21, provided are a CCD drive circuit 47 to output a CCD drive pulse, a CDS/AGC circuit 48 to perform correlated-double sampling and auto-gain control on the output signal from the CCD 21, an A/D converter 49, a DSP (digital-signal processor) 50 to perform various ones of video processing, an electronic variable-power (electronic zoom) circuit 51 to perform an electronic enlarging process sequentially on the video signal outputted from the DSP 50, a D/A converter 52, and a monitor 53 to display thereon a subject-under-observation.

The electronic variable-power circuit 51 is connected with a BPF (band-pass filter) 55 that extracts a high-frequency component of a video signal in order to detect an in-focus status (information) in a video (image). The BPF 5 has an output to be delivered to the auto-focus control section 44b. Meanwhile, a power-change switch 57 is provided as a power-change instructing section on which operation can be done in a NEAR (or TELE: enlargement) direction and in a FAR (or WIDE: reduction) direction. The power-change switch 57 is arranged in the Fig. 3 endoscopic operation part 11B so that the operation signal at the power-change switch 57 can be delivered to the control circuit 44. The power-change switch 57 allows for enlarging operation of both power changes while continuously performing a switchover of from optical power change into electronic power change by means of one NEAR(TELE)-direction switch, and for reducing operation of both power changes while continuously performing a switchover of from electronic power change into optical power change by means of one FAR(WIDE)-direction switch.

Namely, the variable-power control section 44a is to grasp the position of the first and second movable lenses 17a, 17b by inputting therein a power-change position signal (lens position signal) from the lens-drive circuit 43. It also performs an optical variable-power operation by supplying a power-change control signal to the lens-drive circuit 43 in accordance with the operation to the power-change switch 57.

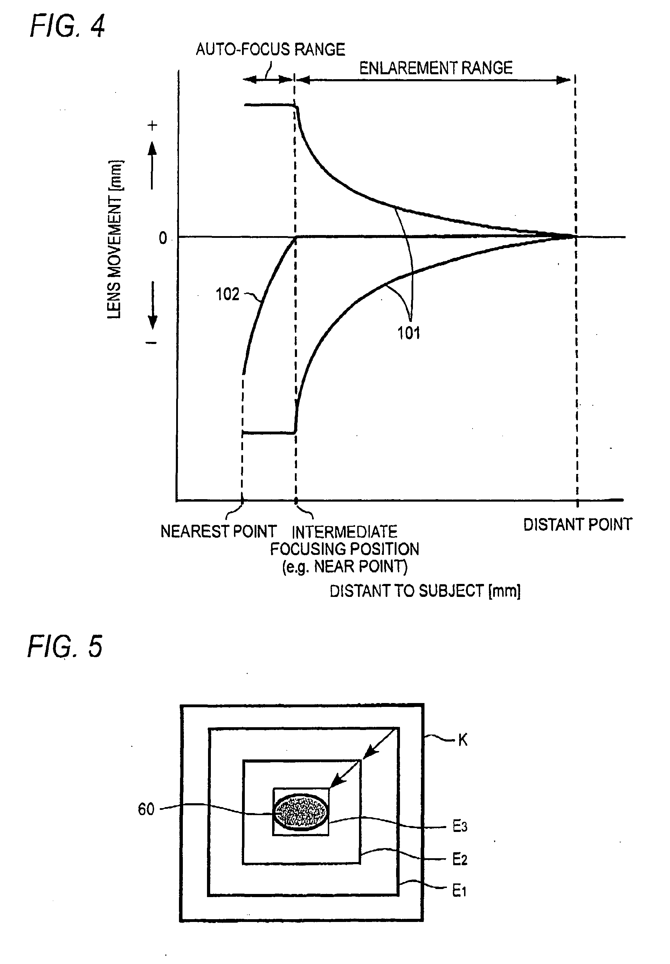

Fig. 4 shows a change of focal point with a change of optical magnification due to a movement of the first to third movable lenses 17a - 17c. As shown in the figure, by moving the power-changing first and second movable lenses 17a, 17b as along the curve line 101 in an enlargement range, the focal point moves from a distant point (FAR end) to, say, a near point (NEAR end) with a change of optical power change. After established at the near point of optical power change, when operation is further made toward NEAR by means of the power-change switch 57, the electronic variable-power circuit 51 performs an electronic power-change processing on the video image in a manner providing a magnification for the display monitor in a continuous fashion.

Because structured as a variable-focal lens, the endoscope at its insertion-part tip surface is placed in a near state to the subject, at the near point (NEAR end). Usually, when conduction an observation with magnification, a hood member is attached at the insertion-part tip of the endoscope so that the hood can be put under light pressure on the subject in a near state. By keeping constant the distance between the insertion-part tip surface of the endoscope and the subject, a first focusing point of the near point is maintained.

However, even in case the hood is put under light pressure on the subject, there encounters a state that the distance is reduced between the insertion-part tip surface of the endoscope and the subject by the effect of pulsation thus causing a blur. Even after established at the near point of optical power change, when further operated toward NEAR by means of the power-change switch 57, the electronic power-change circuit 51 makes an electronic power-change processing of the video image in a manner providing a continuous magnification on the monitor.

Here, in case a blur occurs, the blur is conspicuous. Hence, focal adjustment is performed as in the following.

Meanwhile, the auto-focus control section 44b is to grasp the position of the third movable lens 17c by inputting therein a focus-position signal (lens position signal) from the lens drive circuit 43. During the electronic power-change operation, it performs an auto-focus operation with the third movable lens 17c by supplying the lens drive circuit 43 with a focus control signal generated based upon in-focus state (high-frequency component) signal outputted from the BPF 55. Namely, as shown in Fig. 4, by moving the third movable lens 17c in the optical-axis direction in an auto-focus range, the focal point moves from an intermediate focusing position (according to the first focusing point) up to the nearest position (according to the second focusing point) as shown by the curve line 102, thus making a focal adjustment to the subject.

Fig. 5 shows a setup status of focus-detecting areas within an image area, where positioning is made based on optical power change upon a shift to electronic power change. The auto-focus control section 44b takes size-control of the focus-detecting area for extracting a signal determining whether or not in a focal state, in accordance with a magnification of electronic power change. In the embodiment, as shown in Fig. 5, when the optical power-change position reaches, say, a near point (intermediate focusing position) into a shift to electronic power change, a focus-detecting area E₁ is set up in a size for starting electronic power change relative to the image area (image area by the CCD 21) based on the angle-of-view given by the first and second movable lenses 17a, 17b. Then, focus-detecting areas E₂, E₃ are set up in sizes smaller as the magnification of electronic power change increases. Namely, even where electronically enlarged, in case the detecting area is kept as E₁, focus detection would be done also in the peripheral region other than the enlarged video image to be displayed on the monitor 53, thus failing to perform the optimal focusing on the enlarged video image. By reducing the detecting area depending upon the magnification of electronic enlargement, focal adjustment can be optimally made for the image to be displayed on the monitor 53.

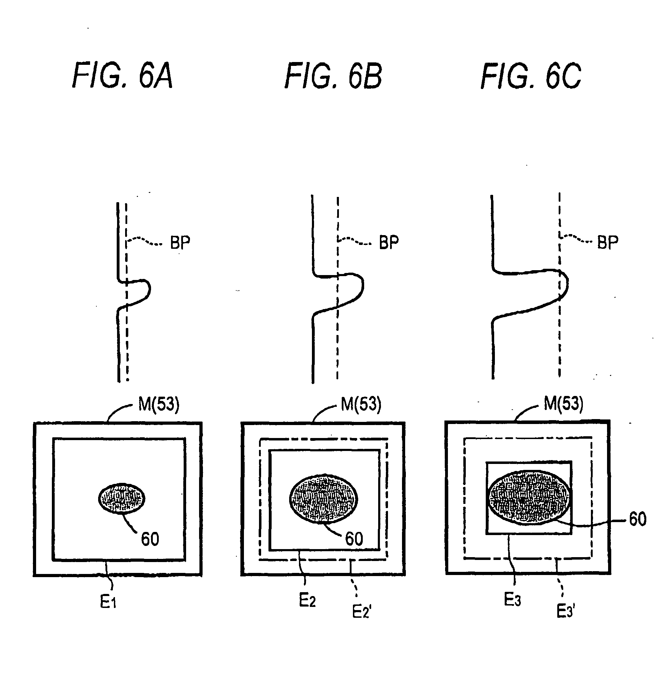

Figs. 6A to 6C show a relationship between the detecting areas E₁-E₃ and the best in-focus point. Fig. 6A shows a case that focus detection is made on the video image in the detecting area E₁, wherein, at a near point, the detecting area E₁ is positioned relative to a display area (screen area) M of the monitor 53 similarly to the foregoing case of the Fig. 5 image area K. The best focus BP, under auto-focusing with the detecting area E₁, is provided in a position, say, of a polyp 60 at its base side thereof (in a position close to a digestive-organ wall), as shown by the dotted line. Contrary to this, when auto-focus is made with the detecting area E₂ smaller than E₁, the best focus BP (dotted line) is given in an intermediate position in the height direction of the polyp 60 as shown in Fig. 6B. When auto-focus is made with the detecting area E₃ further smaller, the best focus BP (dotted line) is given in an upper part of the polyp 60 as shown in Fig. 6C.

In the Figs. 6A to 6C example, the focus-detecting areas E₁ - E₃ were provided smaller as magnification increases, also in the image display area M of the monitor 53 under electronic enlargement. Alternatively, the detecting area can be provided equal in size (smaller relative to the image area K), in each enlargement-video-image display area M that magnification is increased, as shown by E₂', E₃' in Figs. 6B and 6C.

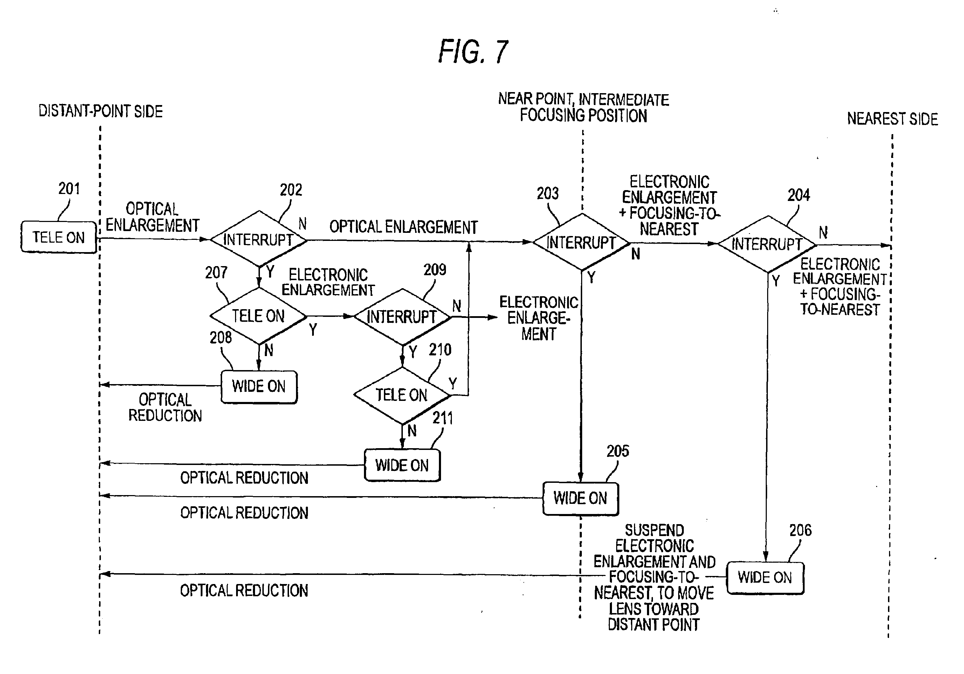

The embodiment is structured as described so far, the operation of which is explained based on Fig. 7. In use of the electronic endoscope 11, when turning on the NEAR-direction switch of the power-change switch 57 arranged on the operation part 11B (step 201), optical power change is effected from a distant (FAR end) side by the first and second movable lenses 17a, 17b. When N (No) at steps 202 and 203 for determining whether to interrupt or not, electronic enlarging operation is made by the electronic variable-power circuit 51 in a continuous fashion (transited automatically) after the first and second movable lenses 17a, 17b in power change reached a near point (intermediate focusing position). In the electronic enlargement (power change), auto-focus (nearest focusing) is effected in the range of from the intermediate focusing position to the nearest point.

Namely, in the optical variable-power section, optical magnification is varied by moving the first and second movable lenses 17a, 17b from a distant point to a near, intermediate focusing position, as shown by the curve line 101. When the switch is operated toward enlargement beyond the optical variable-power range, electronic enlargement is effected. In the electronic enlargement, the third movable lens 17c is moved from the intermediate focusing position up to the extreme proximal point. By changing the focal point as shown by the curve line 102, focal adjustment is performed for the subject. Such auto-focus control for electronic enlargement is implemented by extracting, by the BPF 55, a high-frequency component representative of in-focus state (clarity) from an enlarged video image signal sequentially updated and formed in the electronic variable-power circuit 51 depending upon the output signal from the CCD 21, and supplying, by the focus control section 44b, a focus control signal to the lens drive circuit 43 depending upon the focus-state signal.

Meanwhile, the embodiment sets up focus-detecting areas E₁- E₃ provided smaller as electronic magnification increases as explained in Figs. 5 and 6A to 6C. Accordingly, the state changes from focusing (best focusing BP exists) on a subject to observe, e.g. the polyp 60 and its periphery, as in Fig. 6A, to focusing to the magnified polyp 60 itself, as in Figs. 6B and 6C, thus making it possible to observe a magnifying object in a well state.

In Fig. 7, in case the power-change switch 57 is interrupted (Y) by turning off the NEAR-direction switch at the step 203 and then the FAR-direction switch is turned on (step 205), optical reduction is effected by driving the first and second movable lenses 17a, 17b in a direction of from a near point to a distant point. Meanwhile, in case the power-change switch 57 is similarly interrupted (Y) at step 204 and the FAR-direction switch is turned on (step 206), electric enlargement and auto-focusing (focusing at the nearest) are suspended, to effect optical reduction due to driving the first and second movable lenses 17a, 17b in the direction of from a near point to a distant point.

Meanwhile, in the embodiment, in the case the NEAR-direction switch of the power-change switch 57 in optical enlargement is once suspended from being turned on as in step S202 and further the TELE-direction switch is turned on (step 207), the process transits to electronic enlargement. In the electronic enlargement, in case the TELE-direction switch is once suspended from being turned on as in step 209 and further the TELE-direction switch is turned on (step 210), the process returns to optical enlargement. Incidentally, in the case that determination is N at the steps 207 and 210 and the WIDE-direction switch is turned on, optical reduction is effected. Meanwhile, the switch 57 on-off operating for finely adjusting optical and electronic magnification and the operation instructing for shifting of between electronic enlargement and optical power change, can be distinguished and controlled based on the time length of switch interruption.

In the auto-focus control in the embodiment, explanation was on the case that the intermediate focusing position was provided by the near point. However, as in the case interrupted at step 207 in Fig. 7, there is a possible case that the process transits to electronic power change immediately in front of the near point. In this case, the position immediately in front of the near point may be taken as an intermediate focusing position so that focusing can be made in the range of between the intermediate focusing position and nearest point by the third movable lens 17c.

According to the endoscopic apparatus of the invention, even when further electronically enlarging a video image taken optically at a great magnification, focal adjustment can be done with precision, thus providing an effect that an electronically enlarged video image of a subject can be observed in a well state. Meanwhile, according to the third aspect of the invention, in-focus state can be detected in an area adapted for an enlarged video image for display, thus providing an effect that precise auto-focus is to be done favorably.

The entire disclosure of each and every foreign patent application from which the benefit of foreign priority has been claimed in the present application is incorporated herein by reference, as if fully set forth.

## Claims

1. An endoscopic apparatus comprising an endoscope, the endoscope comprising:
an objective optical system, provided in a distal end of the endoscope, comprising a power-changing movable lens and a focusing movable lens so as to form a subject image; and
an imager that photoelectrically converts the subject image,
so that the endoscopic apparatus has an optical variable-power function that optically changes a magnification of the subject image through the power-changing movable lens and an electronic variable-power function that electronically enlarges the subject image taken by the imager,
wherein the focusing movable lens makes a focal adjustment of the subject image at between: a first focusing point on a near point side from a distant point, the first focusing point being established on the power-changing movable lens having the optical variable-power function; and a second focusing point on a nearest point side, and
wherein the endoscopic apparatus further comprises an auto-focus circuit that performs, during operation with the electronic variable-power function, an auto-focusing on the subject image through use of the focusing movable lens by taking, as the first focusing point, a position of the power-changing movable lens upon shifting from optical power change to electronic power change.

2. An endoscopic apparatus according to claim 1,
wherein the endoscope further comprises an endoscopic operation part including a power-change instructing section that instructs a power change to continuously perform a power change while switching over between the optical variable-power function and the electronic variable-power function, so as to perform an auto-focusing during operation with the electronic variable-power function.

3. An endoscopic apparatus according to claim 1,
wherein the auto-focus circuit establishes focus-detection areas smaller in area correspondingly to an increasing magnification of electronic power change.
